(19) Europäisches Patentamt
European Patent Office
Office européen des brevets

(11) Veröffentlichungsnummer: **0 316 663 A2**

(12) **EUROPÄISCHE PATENTANMELDUNG**

(21) Anmeldenummer: **88118290.1**

(22) Anmeldetag: **03.11.88**

(51) Int. Cl.⁴: **C07D 239/26 , C07D 405/04 , A01N 43/54**

(30) Priorität: **14.11.87 DE 3738715**

(43) Veröffentlichungstag der Anmeldung: **24.05.89 Patentblatt 89/21**

(84) Benannte Vertragsstaaten: **BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnoeckel 59**
**D-5600 Wuppertal 11(DE)**
Erfinder: **Holmwood, Graham, Dr.**
**Krutscheider Weg 105**
**D-5600 Wuppertal 11(DE)**
Erfinder: **Brandes, Wilhelm, Dr.**
**Eichendorffstrasse 3**
**D-5653 Leichlingen 1(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**

(54) **Substituierte Pyrimidin-Derivate.**

(57) Substituierte Pyrimidin-Derivate der Formel (I),

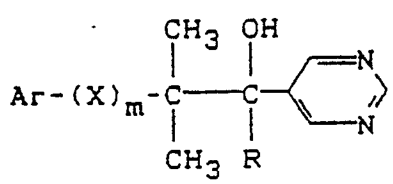

(I)

in welcher
Ar, X, R und m die in der Beschreibung gegebenen Bedeutungen haben, die als Ausgangsverbindungen eingesetzten Pyrimidinyloxirane der Formel (VI) und die Verwendung der Verbindungen der Formel (I) zur Bekämpfung von Schädlingen.

Die substituierten Pyrimidin-Derivate der Formel (I) können z.B. hergestellt werden, indem man geeignete Ketone mit Lithiumpyrimidin oder geeignete Pyrimidinylketone mit Grignard-Verbindungen umsetzt oder geeignete Pyrimidinyloxirane mit Alkoholen der Thiolen umsetzt.

Die neuen Ausgangsverbindungen der Formel (VI) werden aus geeigneten Pyrimidinylketonen durch Umset-

EP 0 316 663 A2

zung mit Dimethyloxosulfoniummethylid oder Trimethylsulfoniummethylsulfat erhalten.

## Substituierte Pyrimidin-Derivate

Die Erfindung betrifft neue substituierte Pyrimidin-Derivate, mehrere Verfahren zu ihrer Herstellung und ihre Verwendung in Schädlingsbekämpfungsmitteln.

Es ist bereits bekannt, daß bestimmte substituierte Pyrimidin-Derivate, wie beispielsweise das 1-(4-Chlorphenoxy)-3,3-dimethyl-2-(5-pyrimidinyl)-butan-2-ol oder das 1-(4-Chlorphenoxy)-3-methyl-2-(5-pyrimidinyl)-butan-2-ol gute fungizide Eigenschaften besitzen (vgl. z.B. EP 1399 oder EP 131 867).

Weiterhin sind substituierte Pyrimidin-Derivate, die eine Wirkung gegen phytopathogene Bakterien und Pilze besitzen, beschrieben in EP 221.014.

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue substituierte Pyrimidin-Derivate der allgemeinen Formel (I),

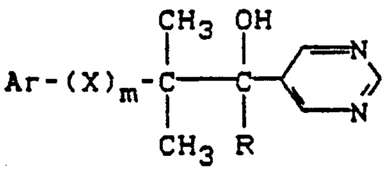

(I)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2$ steht,
wobei
n jeweils für eine Zahl 0, 1 oder 2 steht,
R für Alkyl, Alkenyl, Alkinyl, Alkoxyalkyl, Alkylthioalkyl, Cycloalkyl, Cycloalkylalkyl oder für jeweils gegebenenfalls substituiertes Aryl, Aralkyl, Aryloxyalkyl oder Arylthioalkyl steht und
m für eine Zahl 0 oder 1 steht,
sowie deren pflanzenverträgliche Säureadditionssalze und Metallsalzkomplexe gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyrimidin-Derivate der allgemeinen Formel (I),

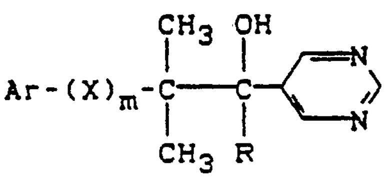

(I)

in welcher
Ar für gegebenenfalls substituiertes Aryl steht,
X für Sauerstoff, Schwefel, Sulfinyl, Sulfinyl, Sulfonyl oder für eine der Gruppen $-CH_2-$; $-O-CH_2-$; $-CH_2-O-$; $-O-CH_2-CH_2$; $-S(O)_n-CH_2-$; $-CH_2-S(O)_n-$ oder $-S(O)_n-CH_2-CH_2$ steht,
wobei
n jeweils für eine Zahl 0, 1 oder 2 steht,
R für Alkyl, Alkenyl, Alkinyl, Alkoxyalkyl, Alkylthioalkyl, Cycloalkyl, Cycloalkylalkyl oder für jeweils gegebenenfalls substituiertes Aryl, Aralkyl, Aryloxyalkyl oder Arylthioalkyl steht und
m für eine Zahl 0 oder 1 steht,
sowie deren pflanzenverträgliche Säureadditionssalze und Metallsalzkomplexe erhält, wenn man

(a) Ketone der Formel (II),

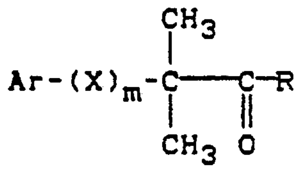

(II)

in welcher
Ar, X, R und m die oben angegebene Bedeutung haben,
mit 5-Lithiumpyrimidin der Formel (III)

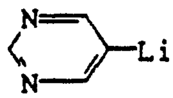

(III)

gegebenenfalls in Gegenwart eines Verdünnungsmittels umsetzt oder wenn man

(b) Pyrimidinylketone der Formel (IV),

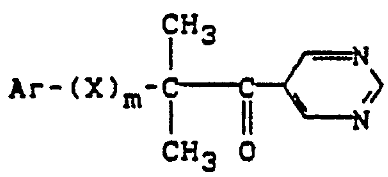

(IV)

in welcher
Ar, X und m die oben angegebene Bedeutung haben,
mit Alkyl-Grignard-Verbindungen der Formel (V),

R-Mg-Hal (V)

in welcher
R die oben angegebene Bedeutung hat und
Hal für Halogen steht,
gegebenenfalls in Gegenwart eines Verdünnungsmittels umsetzt oder wenn man alternativ auch, um substituierte Pyrimidin-Derivate der Formel (Ia),

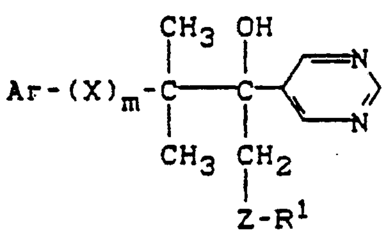

(Ia)

in welcher
Z für Sauerstoff oder Schwefel steht,
$R^1$ für Alkyl oder für gegebenenfalls substituiertes Aryl steht und
Ar, X und m die oben angegebenen Bedeutungen haben,
zu erhalten,

(c) Pyrimidinyloxirane der Formel (VI),

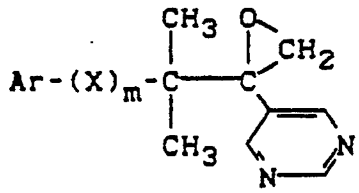

(VI)

in welcher
Ar, X und m die oben angegebene Bedeutung haben,
mit Alkoholen oder Thiolen der Formel (VII),
$R^1$-ZH (VII)
in welcher
$R^1$ und Z die oben angegebene Bedeutung haben,
gegebenenfalls in Gegenwart eines Verdünnungsmittels und gegebenenfalls in Gegenwart eines Reaktionshilfsmittel umsetzt und gegebenenfalls anschließend eine Säure oder ein Metallsalz addiert.

Schließlich wurde gefunden, daß die neuen substituierten Pyrimidin-Derivate der allgemeinen Formel (I) sowie deren Säureadditionssalze und Metallsalzkomplexe eine gute Wirksamkeit gegen Schädlinge insbesondere gegen pilzliche Schädlinge besitzen.

Überraschenderweise zeigen die erfindungsgemäßen substituierten Pyrimidin-Derivate der allgemeinen Formel (I) eine erheblich bessere fungizide Wirksamkeit als z.B. die aus dem Stand der Technik bekannten substituierten Pyrimidin-Derivate, wie das 1-(4-Chlorphenoxy)-3,3-dimethyl-2-(5-pyrimidinyl)-butan-2-ol oder das 1-(4-Chlorphenoxy)-3-methyl-2-(5-pyrimidinyl)-butan-2-ol, welches chemisch und wirkungsmäßig naheliegende Verbindungen sind.

Die erfindungsgemäßen substituierten Pyrimidin-Derivate sind durch die Formel (I) allgemein definiert.
Bevorzugt sind Verbindungen der Formel (I), bei welchen
Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy;
oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;
X für Sauerstoff, Schwefel, Sulfinyl, Sulfonyl oder für eine der Gruppen $-CH_2-$; $-O-CH_2-$; $-CH_2-O-$; $-O-CH_2-CH_2$; $-S(O)_n-CH_2-$; $-CH_2-S(O)_n-$ oder $-S(O)_n-CH_2-CH_2-$ steht,
wobei
n jeweils für eine Zahl 0, 1 oder 2 steht,
R für jeweils geradkettiges oder verzweigtes Alkyl mit 1 bis 6 Kohlenstoffatomen, Alkenyl mit 3 bis 5 Kohlenstoffatomen oder Alkinyl mit 3 bis 5 Kohlenstoffatomen, für jeweils geradkettiges oder verzweigtes Alkoxyalkyl oder Alkylthioalkyl mit jeweils 1 bis 6 Kohlenstoffatomen im Alkoxy- bzw. Alkylthioteil und jeweils 1 bis 3, insbesondere 1 Kohlenstoffatom im Alkylteil, für Cycloalkyl mit 3 bis 7 Kohlenstoffatomen oder für Cycloalkylalkyl mit 3 bis 7 Kohlenstoffatomen im Cycloalkylteil und 1 bis 3 Kohlenstoffatomen im geradkettigen oder verzweigten Alkylteil oder für jeweils gegebenenfalls einfach bis mehrfach, gleich oder verschieden substituiertes Phenyl, Benzyl, Phenylethyl, Phenoxymethyl oder Phenylthiomethyl steht, wobei als Phenylsubstituenten jeweils die bei dem Rest Ar genannten infrage kommen und
m für eine Zahl 0 oder 1 steht.

Besonders bevorzugt sind substituierte Pyrimidin-Derivate der Formel (I), bei welchen
Ar für gegebenenfalls ein- bis dreifach, gleich oder verschieden substituiertes Phenyl steht, wobei als Substituenten infrage kommen; Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen oder jeweils gegebenenfalls ein- bis dreifach, gleich oder verschieden durch Fluor oder Chlor substituiertes Phenyl oder Phenoxy;

oder für gegebenenfalls ein- bis dreifach, gleich oder verschieden substituiertes α-Naphthyl oder β-Naphthyl steht, wobei als Substituenten jeweils infrage kommen: Fluor, Chlor, Brom, Cyano, Methyl, Ethyl, n- oder i-Propyl;

X für Sauerstoff, Schwefel, Sulfinyl, Sulfonyl oder für eine der Gruppen $-CH_2-$; $-O-CH_2-$; $-CH_2-O-$; $-O-CH_2-CH_2$; $-S(O)_n-CH_2-$; $-CH_2-S(O)_n-$ oder $-S(O)_n-CH_2-CH_2$ steht,

wobei

n jeweils für eine Zahl 0, 1 oder 2 steht,

R für Methyl, Ethyl, n- oder i-Propyl, n-, i-, s-oder t-Butyl, Allyl, Propargyl, für jeweils geradkettiges oder verzweigtes Alkoxymethyl oder Alkylthiomethyl mit jeweils 1 bis 4 Kohlenstoffatomen im Alkylteil, für Cyclopropyl, Cyclopentyl, Cyclohexyl, für Cyclopropylmethyl, Cyclopentylmethyl, Cyclohexylmethyl, Cyclopropylethyl, Cyclopentylethyl, Cyclohexylethyl oder für jeweils gegebenenfalls ein- bis dreifach, gleich oder verschieden substituiertes Phenyl, Benzyl, Phenylethyl, Phenoxymethyl oder Phenylthiomethyl steht, wobei als Phenylsubstituenten jeweils die bei dem Rest Ar genannten infrage kommen und

m für eine Zahl 0 oder 1 steht.

Bevorzugte erfindungsgemäße Verbindungen sind auch Additionsprodukte aus Säuren und denjenigen substituierten Pyrimidin-Derivaten der Formel (I), in denen die Substituenten Ar, X, m und R die Bedeutung haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure oder 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Pyrimidin-Derivaten der Formel (I), in denen die Substituenten Ar, X, m und R die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden substituierten Pyrimidin-Derivate der allgemeinen Formel (I) genannt:

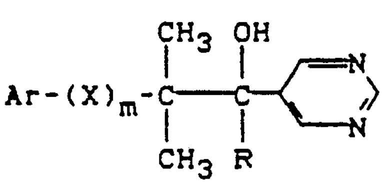

(I)

| Ar | $-(X)_m-$ | R |
| --- | --- | --- |
| 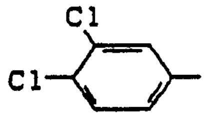 | $-CH_2-$ | $CH_3$ |
| 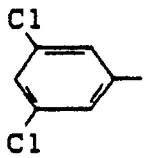 | $-CH_2-$ | $CH_3$ |
| 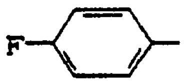 | $-CH_2-$ | $CH_3$ |
| 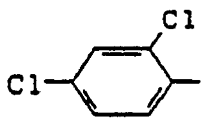 | $-CH_2-$ | $CH_3$ |
| 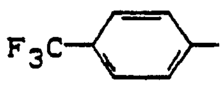 | $-CH_2-$ | $CH_3$ |
| 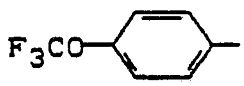 | $-CH_2-$ | $CH_3$ |
| 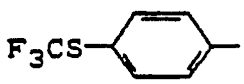 | $-CH_2-$ | $CH_3$ |
| 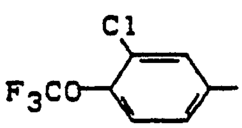 | $-CH_2-$ | $CH_3$ |

| Ar | $-(X)_m-$ | R |
|---|---|---|
| 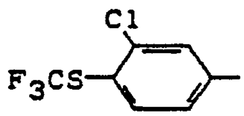 | $-CH_2-$ | $CH_3$ |
| 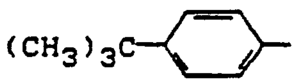 | $-CH_2-$ | $CH_3$ |
| 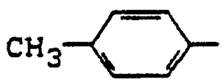 | $-CH_2-$ | $CH_3$ |
| 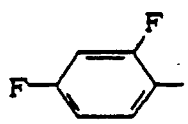 | $-CH_2-$ | $CH_3$ |
| 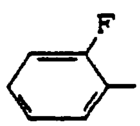 | $-CH_2-$ | $CH_3$ |
| 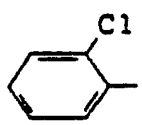 | $-CH_2-$ | $CH_3$ |
| 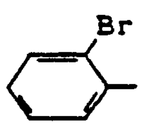 | $-CH_2-$ | $CH_3$ |
| 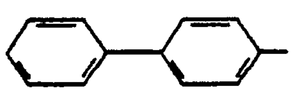 | $-CH_2-$ | $CH_3$ |

| Ar | $-(X)_m-$ | R |
|---|---|---|
| 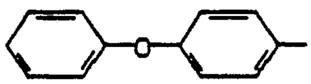 | $-CH_2-$ | $CH_3$ |
| 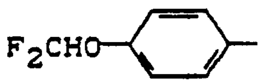 | $-CH_2-$ | $CH_3$ |
| 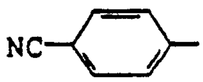 | $-CH_2-$ | $CH_3$ |
| 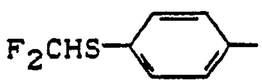 | $-CH_2-$ | $CH_3$ |
| 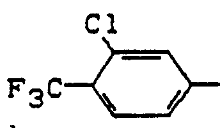 | $-CH_2-$ | $CH_3$ |
| 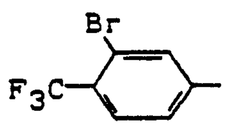 | $-CH_2-$ | $CH_3$ |
| 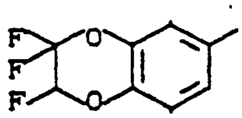 | $-CH_2-$ | $CH_3$ |
| 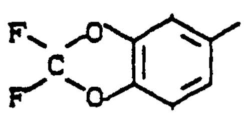 | $-CH_2-$ | $CH_3$ |

| Ar | $-(X)_m-$ | R |
|---|---|---|
| 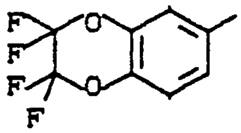 | $-CH_2-$ | $CH_3$ |
| 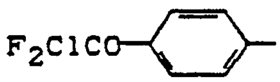 | $-CH_2-$ | $CH_3$ |
| 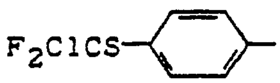 | $-CH_2-$ | $CH_3$ |
| 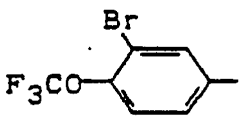 | $-CH_2-$ | $CH_3$ |
| 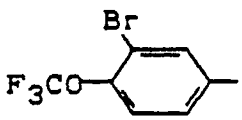 | $-CH_2-$ | $CH_3$ |
| 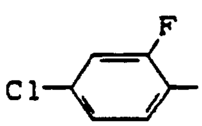 | $-CH_2-$ | $CH_3$ |
| 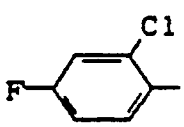 | $-CH_2-$ | $CH_3$ |

| Ar | $-(X)_m-$ | R |
| --- | --- | --- |
| 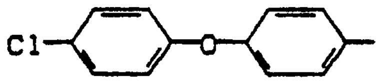 | $-CH_2-$ | $CH_3$ |
| 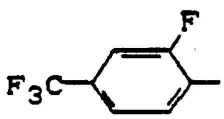 | $-CH_2-$ | $CH_3$ |
| 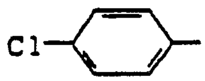 | $-O-CH_2-$ | $CH_3$ |
| 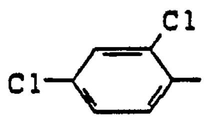 | $-O-CH_2-$ | $CH_3$ |
| 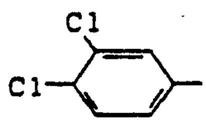 | $-O-CH_2-$ | $CH_3$ |
| 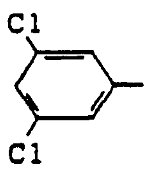 | $-O-CH_2-$ | $CH_3$ |
| 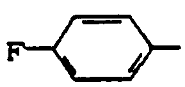 | $-O-CH_2-$ | $CH_3$ |
| 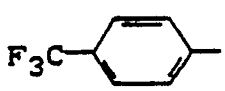 | $-O-CH_2-$ | $CH_3$ |

| Ar | $-(X)_m-$ | R |
|---|---|---|
| 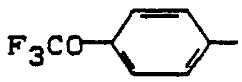 | $-O-CH_2-$ | $CH_3$ |
| 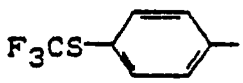 | $-O-CH_2-$ | $CH_3$ |
| 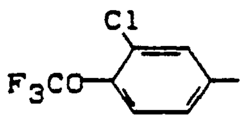 | $-O-CH_2-$ | $CH_3$ |
| 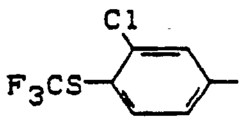 | $-O-CH_2-$ | $CH_3$ |
| 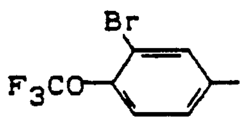 | $-O-CH_2-$ | $CH_3$ |
| 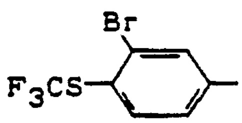 | $-O-CH_2-$ | $CH_3$ |
| 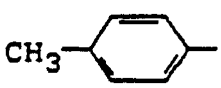 | $-O-CH_2-$ | $CH_3$ |
| 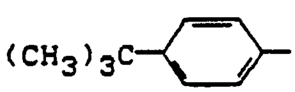 | $-O-CH_2-$ | $CH_3$ |

| Ar | $-(X)_m-$ | R |
|---|---|---|
| 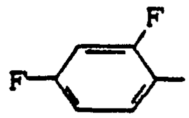 | $-O-CH_2-$ | $CH_3$ |
| 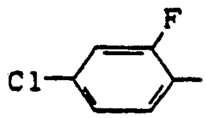 | $-O-CH_2-$ | $CH_3$ |
| 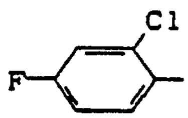 | $-O-CH_2-$ | $CH_3$ |
| 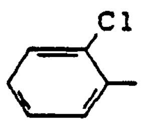 | $-O-CH_2-$ | $CH_3$ |
| 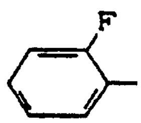 | $-O-CH_2-$ | $CH_3$ |
| 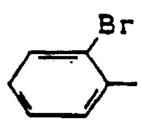 | $-O-CH_2-$ | $CH_3$ |
| 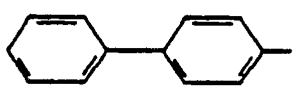 | $-O-CH_2-$ | $CH_3$ |

| Ar | $-(X)_m-$ | R |
|---|---|---|
| 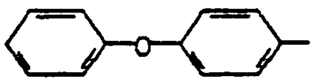 | $-O-CH_2-$ | $CH_3$ |
| 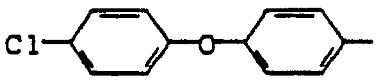 | $-O-CH_2-$ | $CH_3$ |
| 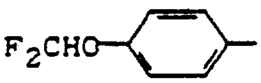 | $-O-CH_2-$ | $CH_3$ |
| 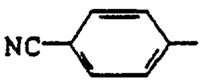 | $-O-CH_2-$ | $CH_3$ |
| 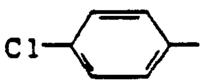 | $-S-CH_2-$ | $CH_3$ |
| 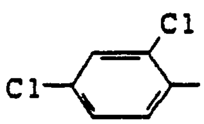 | $-S-CH_2-$ | $CH_3$ |
| 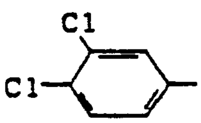 | $-S-CH_2-$ | $CH_3$ |
| 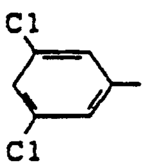 | $-S-CH_2-$ | $CH_3$ |

| Ar | $-(X)_m-$ | R |
|---|---|---|
| 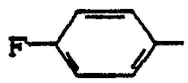 | $-S-CH_2-$ | $CH_3$ |
| 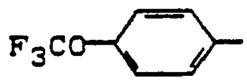 | $-S-CH_2-$ | $CH_3$ |
| 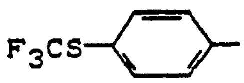 | $-S-CH_2-$ | $CH_3$ |
| 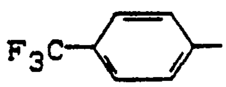 | $-S-CH_2-$ | $CH_3$ |
| 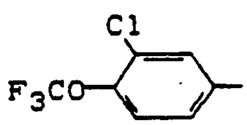 | $-S-CH_2-$ | $CH_3$ |
| 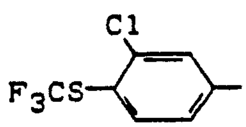 | $-S-CH_2-$ | $CH_3$ |
| 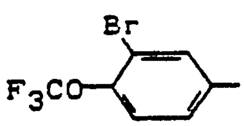 | $-S-CH_2-$ | $CH_3$ |
| 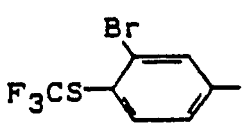 | $-S-CH_2-$ | $CH_3$ |

| Ar | $-(X)_m-$ | R |
|---|---|---|
| 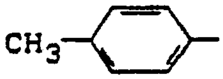 | $-S-CH_2-$ | $CH_3$ |
| 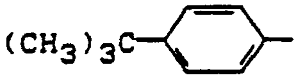 | $-S-CH_2-$ | $CH_3$ |
| 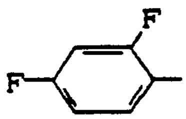 | $-S-CH_2-$ | $CH_3$ |
| 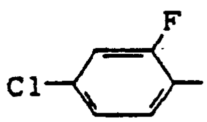 | $-S-CH_2-$ | $CH_3$ |
| 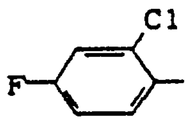 | $-S-CH_2-$ | $CH_3$ |
| 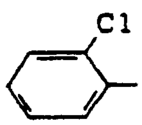 | $-S-CH_2-$ | $CH_3$ |
| 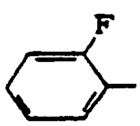 | $-S-CH_2-$ | $CH_3$ |

| Ar | $-(X)_m-$ | R |
|---|---|---|
| 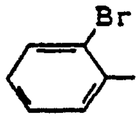 | $-S-CH_2-$ | $CH_3$ |
| 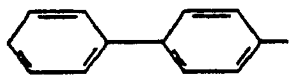 | $-S-CH_2-$ | $CH_3$ |
| 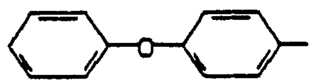 | $-S-CH_2-$ | $CH_3$ |
| 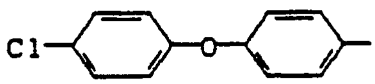 | $-S-CH_2-$ | $CH_3$ |
| 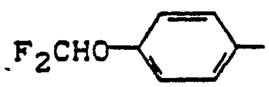 | $-S-CH_2-$ | $CH_3$ |
| 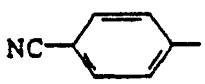 | $-S-CH_2-$ | $CH_3$ |
| 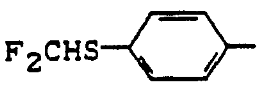 | $-S-CH_2-$ | $CH_3$ |
| 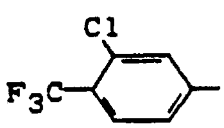 | $-S-CH_2-$ | $CH_3$ |

| Ar | $-(X)_m-$ | R |
|---|---|---|
| 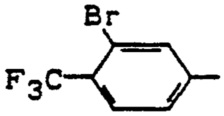 | $-S-CH_2-$ | $CH_3$ |
| 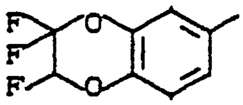 | $-S-CH_2-$ | $CH_3$ |
| 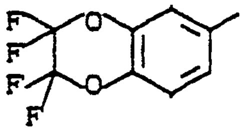 | $-S-CH_2-$ | $CH_3$ |
| 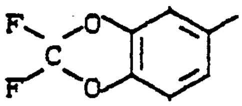 | $-S-CH_2-$ | $CH_3$ |
| 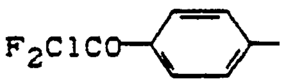 | $-S-CH_2-$ | $CH_3$ |
| 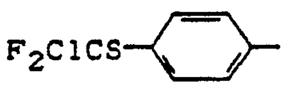 | $-S-CH_2-$ | $CH_3$ |
| 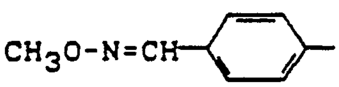 | $-S-CH_2-$ | $CH_3$ |
| 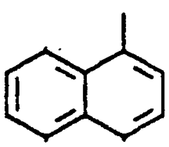 | $-S-CH_2-$ | $CH_3$ |

| Ar | $-(X)_m-$ | R |
|---|---|---|
| 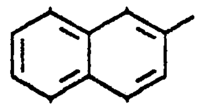 | $-S-CH_2-$ | $CH_3$ |
| 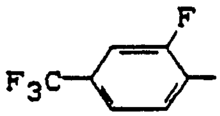 | $-S-CH_2-$ | $CH_3$ |
| 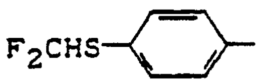 | $-O-CH_2-$ | $CH_3$ |
| 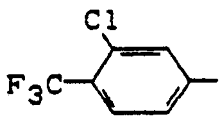 | $-O-CH_2-$ | $CH_3$ |
| 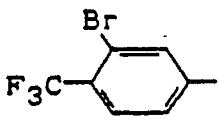 | $-O-CH_2-$ | $CH_3$ |
| 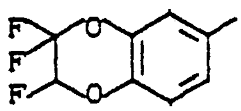 | $-O-CH_2-$ | $CH_3$ |
| 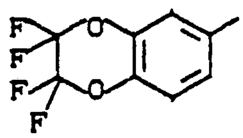 | $-O-CH_2-$ | $CH_3$ |
| 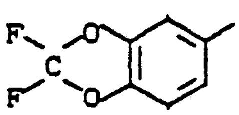 | $-O-CH_2-$ | $CH_3$ |

| Ar | $-(X)_m-$ | R |
|---|---|---|
| 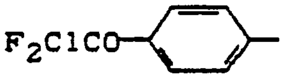 | $-O-CH_2-$ | $CH_3$ |
| 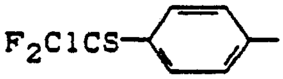 | $-O-CH_2-$ | $CH_3$ |
| 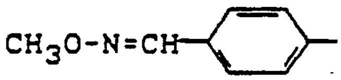 | $-O-CH_2-$ | $CH_3$ |
| 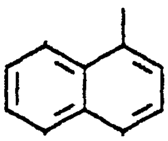 | $-O-CH_2-$ | $CH_3$ |
| 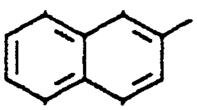 | $-O-CH_2-$ | $CH_3$ |
| 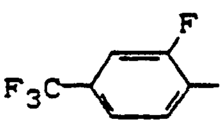 | $-O-CH_2-$ | $CH_3$ |
| 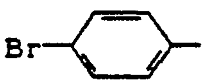 | -O- | $CH_3$ |
| 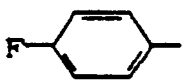 | -O- | $CH_3$ |

| Ar | $-(X)_m-$ | R |
|---|---|---|
| 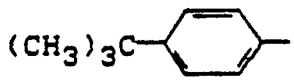 | -O- | $CH_3$ |
| 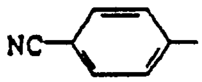 | -O- | $CH_3$ |
| 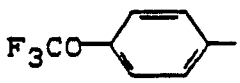 | -O- | $CH_3$ |
| 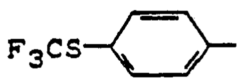 | -O- | $CH_3$ |
| 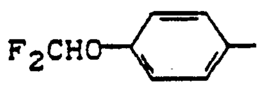 | -O- | $CH_3$ |
| 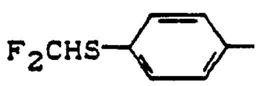 | -O- | $CH_3$ |
| 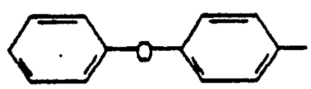 | -O- | $CH_3$ |
| 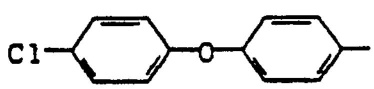 | -O- | $CH_3$ |

| Ar | $-(X)_m-$ | R |
|---|---|---|
| 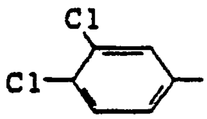 | -O- | $CH_3$ |
| 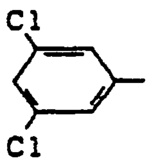 | -O- | $CH_3$ |
| 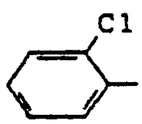 | -O- | $CH_3$ |
| 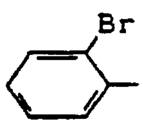 | -O- | $CH_3$ |
| 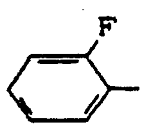 | -O- | $CH_3$ |
| 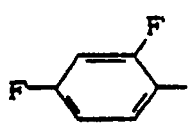 | -O- | $CH_3$ |
| 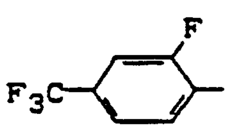 | -O- | $CH_3$ |

| Ar | $-(X)_m-$ | R |
|---|---|---|
| 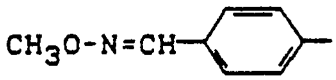 | -O- | $CH_3$ |
| 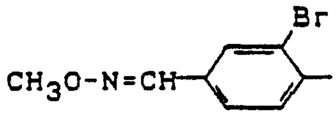 | -O- | $CH_3$ |
| 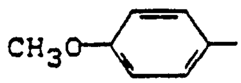 | -O- | $CH_3$ |
| 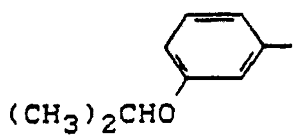 | -O- | $CH_3$ |
| 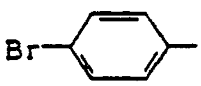 | -S- | $CH_3$ |
| 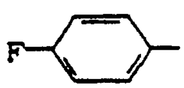 | -S- | $CH_3$ |
| 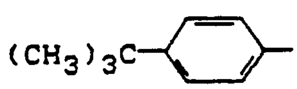 | -S- | $CH_3$ |
| 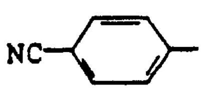 | -S- | $CH_3$ |

| Ar | $-(X)_m-$ | R |
|---|---|---|
| 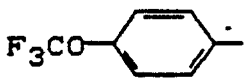 | -S- | $CH_3$ |
| 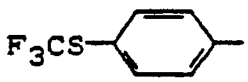 | -S- | $CH_3$ |
| 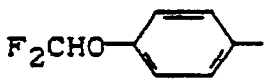 | -S- | $CH_3$ |
| 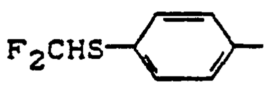 | -S- | $CH_3$ |
| 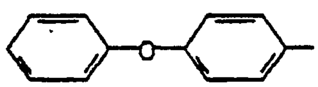 | -S- | $CH_3$ |
| 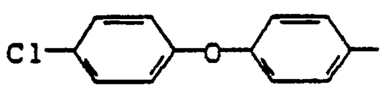 | -S- | $CH_3$ |
| 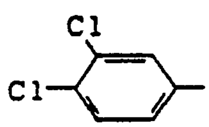 | -S- | $CH_3$ |
| 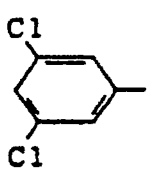 | -S- | $CH_3$ |

| Ar | -(X)$_m$- | R |
|---|---|---|
| 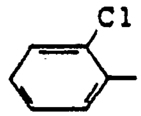 | -S- | $CH_3$ |
| 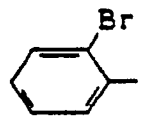 | -S- | $CH_3$ |
| 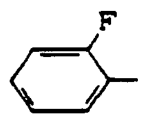 | -S- | $CH_3$ |
| 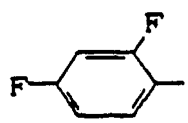 | -S- | $CH_3$ |
| 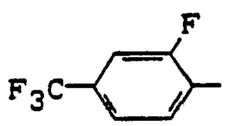 | -S- | $CH_3$ |
| 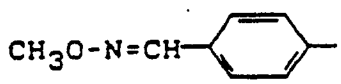 | -S- | $CH_3$ |
| 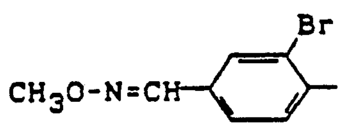 | -S- | $CH_3$ |

| Ar | $-(X)_m-$ | R |
|---|---|---|
| 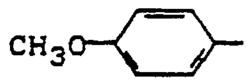 | -S- | $CH_3$ |
| 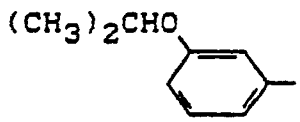 | -S- | $CH_3$ |
| 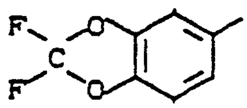 | -S- | $CH_3$ |
| 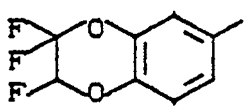 | -S- | $CH_3$ |
| 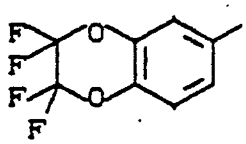 | -S- | $CH_3$ |
| 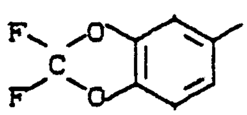 | -O- | $CH_3$ |
| 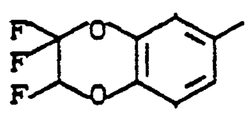 | -O- | $CH_3$ |
| 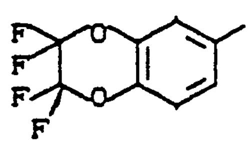 | -O- | $CH_3$ |

| Ar | $-(X)_m-$ | R |
|---|---|---|
| 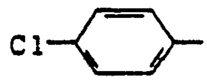 | $-O-CH_2-CH_2-$ | $CH_3$ |
| 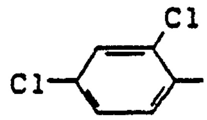 | $-O-CH_2-CH_2-$ | $CH_3$ |
| 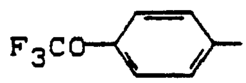 | $-O-CH_2-CH_2-$ | $CH_3$ |
| 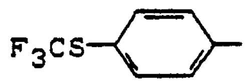 | $-O-CH_2-CH_2-$ | $CH_3$ |
| 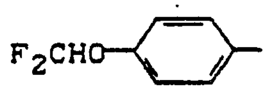 | $-O-CH_2-CH_2-$ | $CH_3$ |
| 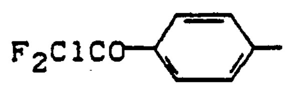 | $-O-CH_2-CH_2-$ | $CH_3$ |
| 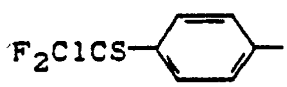 | $-O-CH_2-CH_2-$ | $CH_3$ |
| 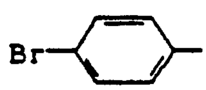 | $-O-CH_2-CH_2-$ | $CH_3$ |

| Ar | $-(X)_m-$ | R |
|---|---|---|
| 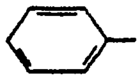 | $-O-CH_2-CH_2-$ | $CH_3$ |
| 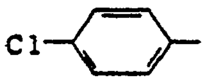 | - | $CH_3$ |
| 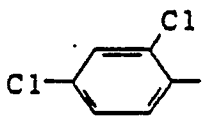 | - | $CH_3$ |
| 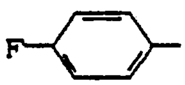 | - | $CH_3$ |
| 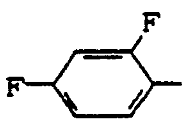 | - | $CH_3$ |
| 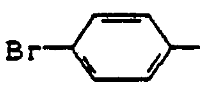 | - | $CH_3$ |
| 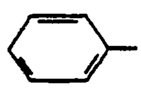 | - | $CH_3$ |
| 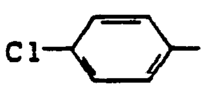 | $-CH_2-$ | 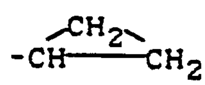 |

| Ar | $-(X)_m-$ | R |
|---|---|---|
| 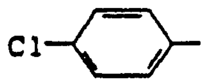 | $-CH_2-$ | 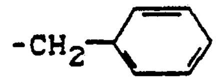 |
| 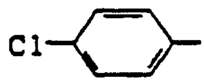 | $-CH_2-$ | 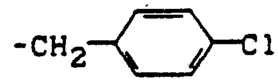 |
| 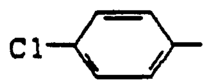 | $-CH_2-$ | $-CH_2-OCH_3$ |
| 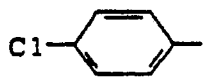 | $-CH_2-$ | $-CH_2-SCH_3$ |
| 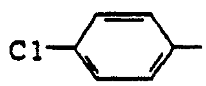 | $-CH_2-$ | 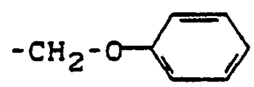 |
| 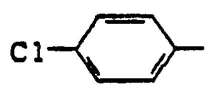 | $-CH_2-$ | 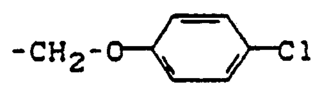 |
| 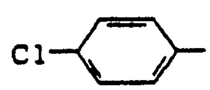 | $-CH_2-$ | 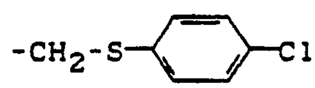 |
| 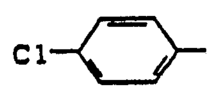 | $-CH_2-$ | $-CH_2-CH=CH_2$ |

| Ar | $-(X)_m-$ | R |
|---|---|---|
| 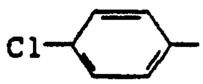 | $-CH_2-$ | $-CH_2-C{\equiv}CH$ |
| 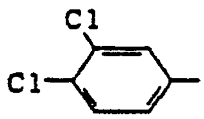 | $-CH_2-$ | $-CH_2-S-CH_3$ |
| 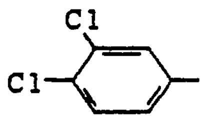 | $-CH_2-$ | $-CH_2-OCH_3$ |
| 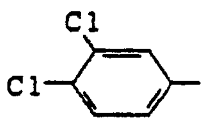 | $-CH_2-$ | $-CH_2-CH{=}CH_2$ |
| 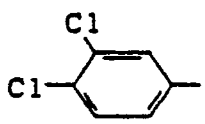 | $-CH_2-$ | $-CH_2-C{\equiv}CH$ |
| 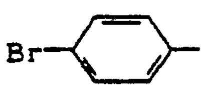 | $-CH_2-$ | $-CH_2-OCH_3$ |
| 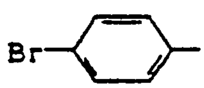 | $-CH_2-$ | $-CH_2-S-CH_3$ |
| 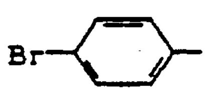 | $-CH_2-$ | $-CH_2-CH{=}CH_2$ |

| Ar | $-(X)_m-$ | R |
|---|---|---|
| 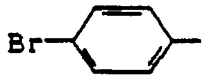 | $-CH_2-$ | $-CH_2-C{\equiv}CH$ |
| 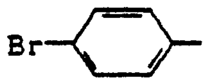 | $-CH_2-$ | 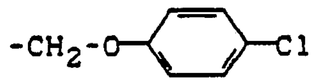 |
| 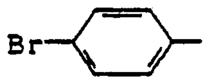 | $-CH_2-$ | 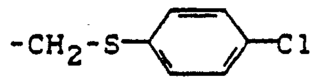 |

Verwendet man beispielsweise 3-(4-Chlorphenoxy)-3-methyl-butan-2-on und 5-Lithiumpyrimidin als Ausgangstoffe, so läßt sich der Reaktionsablauf des erfindungsgemäßen Verfahrens (a) durch das folgende Formelschema darstellen:

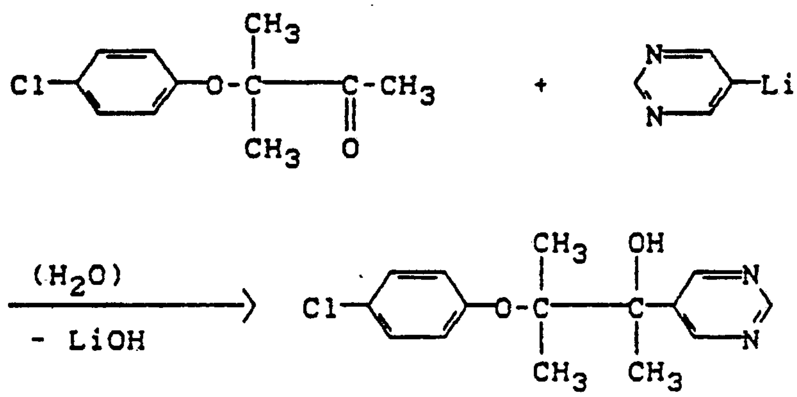


Verwendet man beispielsweise 3-(4-Fluorphenyl)-2,2-dimethyl-1-(5-pyrimidinyl)-propan-1-on und Allylmagnesiumbromid als Ausgangsstoffe, so läßt sich der Reaktionsablauf des erfindungsgemäßen Verfahrens (b) durch das folgende Formelschema darstellen:

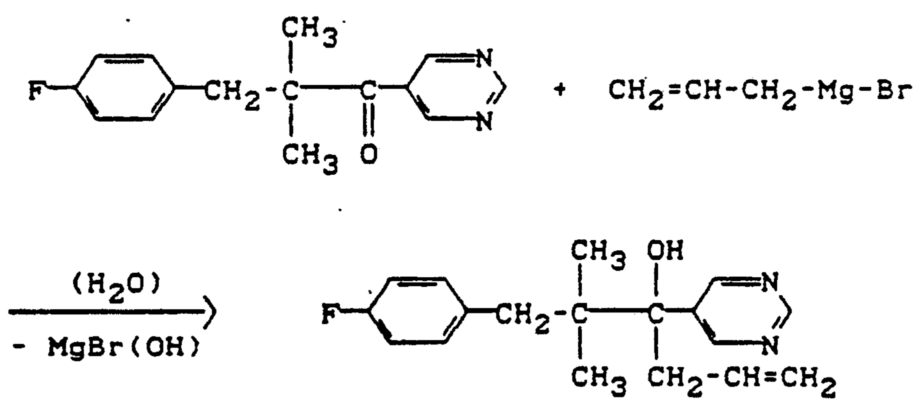


Verwendet man beispielsweise 2-[2-(2,4-Dichlorphenoxy)-2-propyl]-2-(5-pyrimidinyl)-oxiran und 4-Chlorphenol als Ausgangsstoffe, so läßt sich der Reaktionsablauf des erfindungsgemäßen Verfahrens (c)

durch das folgende Formelschema darstellen:

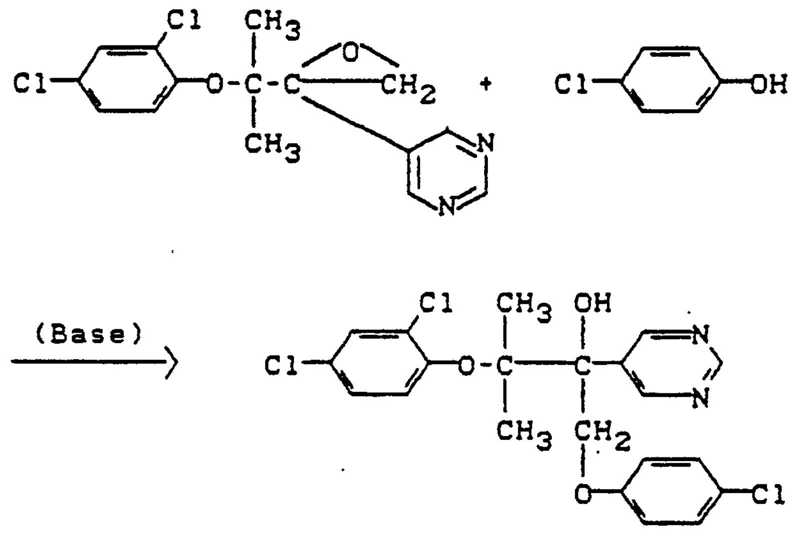


Die zur Durchführung des erfindungsgemäßen Verfahrens (a) als Ausgangsstoffe benötigten Ketone sind durch die formel (II) allgemein definiert. In dieser Formel (II) stehen R, Ar, X und m vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Ketone der Formel (II) sind bekannt oder können in Analogie zu bekannten Verfahren erhalten werden (vgl. z.B. DE-OS 3 210 725 oder DE-OS 3 048 266).

Das zur Durchführung des erfindungsgemäßen Verfahrens (a) weiterhin als Ausgangsverbindung benötigte 5-Lithiumpyrimidin ist ebenfalls bekannt (vgl. z.B. EP 131 867).

Die zur Durchführung des erfindungsgemäßen Verfahrens (b) als Ausgangsstoffe benötigten Pyrimidinylketone sind durch die Formel (IV) allgemein definiert. In dieser Formel (IV) stehen Ar, X und m vorzugsweise für diejenigen Reste, die bereits im Zusammenhang mit der Beschreibung der erfindungsgemäßen Stoffe der Formel (I) als bevorzugt für diese Substituenten genannt wurden.

Die Pyrimidinylketone der Formel (IV) sind bekannt (vgl. EP 273 190, EP 221 014) oder Gegenstand einer eigenen noch nicht publizierten Patentanmeldung (vgl. Deutsche Patentanmeldung P 37 02 962 vom 31.01.1987) und erhältlich in Analogie zu den dort beschriebenen Verfahren.

Die zur Durchführung des erfindungsgemäßen Verfahrens (b) weiterhin als Ausgangsstoffe benötigten Alkyl-Grignard-Verbindungen sind durch die Formel (V) allgemein definiert. In dieser Formel (V) steht R vorzugsweise für diejenigen Reste, die bereits im Zusammenhang mit der Beschreibung der erfindungsgemäßen Stoffe der Formel (I) als bevorzugt für diesen Substituenten genannt wurden.

Hal steht vorzugsweise für Chlor, Brom oder Iod.

Die Alkyl-Grignard-Verbindungen der Formel (V) sind allgemein bekannte Verbindungen der organischen Chemie.

Die zur Durchführung des erfindungsgemäßen Verfahrens (c) als Ausgangsstoffe benötigten Pyrimidinyloxirane sind durch die Formel (VI) allgemein definiert. In dieser Formel (VI) stehen Ar, X und m vorzugsweise für diejenigen Reste und Indices, die bereits im Zusammenhang mit der Beschreibung der erfindungsgemäßen Stoffe der Formel (I) als bevorzugt für diese Substituenten und Indices genannt wurden.

Die Pyrimidinyloxirane der Formel (VI) sind noch nicht bekannt.

Man erhält die Pyrimidinyloxirane der Formel (VI),

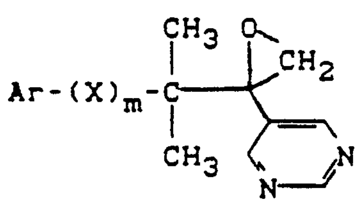

(VI)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,
wobei
n jeweils für eine Zahl 0, 1 oder 2 steht,
und
m für eine Zahl 0 oder 1 steht, wenn man Pyrimidinylketone der Formel (IV)

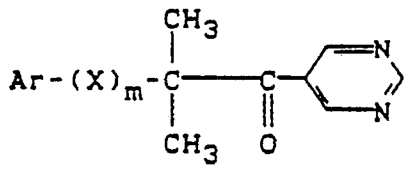
(IV)

in welchen
Ar, X und m die oben angegebene Bedeutung haben, entweder mit Dimethyloxosulfonium-methylid der Formel (X)

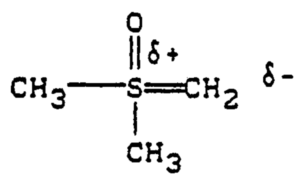
(X)

in bekannter Weise in Gegenwart eines Verdünnungsmittels, wie beispielsweise Dimethylsulfoxid, bei Temperaturen zwischen 20 °C und 80 °C umsetzt (vgl. z.B. J. Amer. Chem. Soc. 87, 1363 - 1364 [1965]), oder mit Trimethylsulfoniummethylsulfat der Formel (XI)

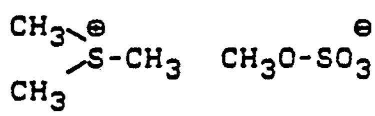
(XI)

in bekannter Weise in Gegenwart eines Verdünnungsmittels, wie beispielsweise Acetonitril und in Gegenwart einer Base wie beispielsweise Natriummethylat, bei Temperaturen zwischen 0 °C und 60 °C umsetzt (vgl. z.B. Heterocycles 8, 397 [1977]).

Die so erhältlichen Pyrimidinyloxirane der Formel (VI) können gegebenenfalls ohne Isolierung direkt aus dem Reaktionsgemisch heraus weiter umgesetzt werden.

Die zur Durchführung des erfindungsgemäßen Verfahrens (c) weiterhin als Ausgangsstoffe benötigten Alkohole oder Thiole sind durch die Formel (VII) allgemein definiert. In dieser Formel (VII) steht $R^1$ vorzugsweise für geradkettiges oder verzweigtes Alkyl mit 1 bis 6 Kohlenstoffatomen oder für gegebenenfalls einfach bis mehrfach, gleich oder verschieden substituiertes Phenyl, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy,
$R^1$ steht besonders bevorzugt für Methyl, Ethyl, n-oder i-Propyl, n-, i-, s- oder t-Butyl oder für gegebenenfalls ein- bis dreifach, gleich oder verschieden substituiertes Phenyl, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen sowie jeweils gegebenenfalls ein-bis dreifach, gleich oder verschieden durch Fluor oder Chlor substituiertes Phenyl oder Phenoxy,

Z steht vorzugsweise für Sauerstoff oder Schwefel.

Die Alkohole oder Thiole der Formel (VII) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Diisopropylether, Dioxan, Tetrahydrofuran oder Ethylenglykoldimethyl- oder -diethylether oder Amide, wie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen - 120 °C und - 60 °C, vorzugsweise bei Temperaturen zwischen - 120 °C und - 80 °C.

Zur Durchführung des erfindungsgemäßen Verfahrens (a) setzt man pro Mol an Keton der Formel (II) im allgemeinen 1.0 bis 3.0 Mol, vorzugsweise 1.0 bis 1.5 Mol an 5-Lithium-pyrimidin der Formel (III) ein.

Die Reaktionsdurchführung erfolgt nach allgemein üblichen Methoden.

Dabei kann die Umsetzung erforderlichenfalls in Gegenwart eines geeigneten Inertgases, wie beispielsweise Stickstoff oder Helium durchgeführt werden. Es ist auch möglich, das als Reaktionspartner eingesetzte 5-Lithium-pyrimidin der Formel (III) aus geeigneten Ausgangsverbindungen, wie beispielsweise 5-Brompyrimidin und n-Buthyllithium, in einer vorgelagerten Reaktion direkt im Reaktionsgefäß herzustellen und ohne Isolierung im Eintopverfahren mit den Ketonen der Formel (II) weiter umzusetzen.

Die Aufarbeitung und Isolierung der Reaktionsprodukte erfolgt ebenfalls nach üblichen Methoden (vgl. die Her stellungsbeispiele).

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel als Verdünnungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen - 50 °C und + 150 °C, vorzugsweise bei Temperaturen zwischen - 20 °C und + 100 °C.

Zur Durchführung des erfindungsgemäßen Verfahrens (b) setzt man pro Mol an Pyrimidinylketon der Formel (IV) im allgemeinen 1.0 bis 3.0 Mol, vorzugsweise 1.0 bis 1.5 Mol an Alkyl-Grignard-Verbindung der Formel (V) ein. Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril oder Amide, wie Dimethylformamid, Dimethylacetamid, N-Methylformanilid, N-Methylpyrrolidon oder Hexamethylphosphorsäuretriamid.

Es ist gegebenenfalls auch möglich, die als Reaktionspartner verwendeten Alkohole oder Thiole der Formel (VII) in entsprechendem Überschuß gleichzeitig als Verdünnungsmittel einzusetzen.

Das erfindungsgemäße Verfahren (c) wird vorzugsweise in Gegenwart eines geeigneten basischen Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, N,N-Dimethyl aminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 200 °C, vorzugsweise bei Temperaturen zwischen 60 °C und 150 °C.

Zur Durchführung des erfindungsgemäßen Verfahrens (c) setzt man pro Mol an Pyrimidinyloxiran der Formel (VI) im allgemeinen 1.0 bis 20.0 Mol, vorzugsweise 1.0 bis 5.0 Mol an Alkohol oder Thiol der Formel (VII) und gegebenenfalls 1.0 bis 5.0 Mol, vorzugsweise 1.0 bis 2.0 Mol an basischem Reaktionshilfsmittel ein.
Die Reaktionsdurchführung, Aufarbeitung und Isolierung der Reaktionsprodukte erfolgt nach allgemein üblichen Methoden (vgl. z.B. DE-OS 3 427 844).

Gegebenenfalls kann man die mit Hilfe der erfindungsgemäßen Verfahen (a), (b) oder (c) erhältlichen substituierten Pyrimidin-Derivate der Formel (I) nach üblichen Methoden derivatisieren, beispielsweise indem man die Hydroxygruppe mit üblichen Alkylierungs- oder Acylierungsmitteln umsetzt. Die so erhältlichen Alkylierungs-oder Acylierungsprodukte der erfindungsgemäßen Verbindungen der Formel (I) besitzen ebenfalls gute fungizide Wirksamkeit.

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Brom wasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.
Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.
Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.
Als Anionen von Salzen kommen solche in Betracht, die sich vorzugsweise von folgenden Säuren ableiten: Halogenwasserstoffsäure, wie z.B. Chlorwasserstoffsäure und Bromwasserstoffsäure, ferner Phosphorsäure, Salpetersäure und Schwefelsäure.
Die Metallsalzkomplexe von Verbindungen der Formel (I) können in einfacher Weise nach üblichen Verfahren er halten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe können für den Gebrauch als Pflanzenschutzmittel insbesondere als Fungizide eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder p. graminea

(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), gegen den Erreger der Braunfleckigkeit des Weizens (Leptosphaeria nodorum), gegen den Erreger der Braunfleckenkrankheit der Gerste (Cochliobolus sativus), gegen den Erreger der Streifenkrankheit der Gerste (Drechslera graminea) oder gegen den Erreger der Blattfleckenkrankheit der Gerste (Pyrenophora teres), zur Bekämpfung von Reiskrankheiten, wie beispielsweise zur Bekämpfung der Reisfleckenkrankheit (Pyricularia oryzae) oder zur Bekämpfung von Krankheiten im Obst- und Gemüsebau, wie beispielsweise gegen den Erreger des Bohnengrauschimmels (Botrytis cinerea) oder gegen den Erreger des Apfelschorfes (Venturia inaequalis) einsetzen.

Darüberhinaus besitzen die erfindungsgemäßen Wirkstoffe eine besonders breite fungizide Wirksamkeit bei in-vitro-Versuchen.

In entsprechenden Aufwandmengen besitzen die erfindungsgemäßen Wirkstoffe darüberhinaus auch eine wachstumsregulierende Wirkung bei Kulturpflanzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

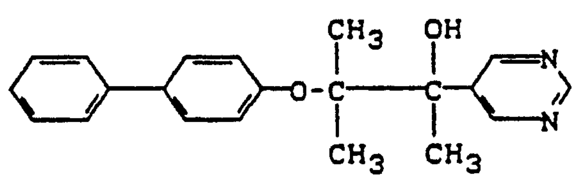

(Verfahren a)

Zu 33,4 g (0,21 Mol) 5-Brompyrimidin in 150 ml absolutem Ether und 150 ml absolutem Tetrahydrofuran gibt man bei - 115 °C unter einem trockenen Stickstoffstrom tropfenweise unter Rühren 150 ml (0.24 Mol) einer 15prozentigen n-Butyllithiumlösung in Hexan, rührt nach beendeter Zugabe eine weitere Stunde bei - 155 °C und gibt dann tropfenweise unter Rühren innerhalb von einer Stunde eine Lösung von 50,86 g (0.2 Mol) 3-[(1,1′-Biphenyl)-4-yloxy]-3-methylbutan-2-on in 45 ml absolutem Ether und 45 ml absolutem Tetrahydrofuran zu, rührt eine weitere Stunde bei - 115 °C und anschließend eine Stunde bei - 70 °C, versetzt dann mit einer Lösung von 15 g Ammoniumchlorid in 75 ml Wasser und läßt die Mischung auf Raumtemperatur kommen. Zur Aufarbeitung wird die wässrige Phase abgetrennt, die organische Phase zweimal mit gesättigter wässriger Kochsalzlösung gewaschen, über Natriumsulfat getrocknet, im Vakuum eingeengt, der ölige Rückstand durch Verrühren mit Dichlormethan/Essigester (2:1) kristallisiert und aus Acetonitril umkristallisiert.

Man erhält 22,9 (34,3 % der Theorie) an 3-[(1,1′-Biphenyl)-4-yloxy]-3-methyl-2-pyrimidin-5-yl-butan-2-ol vom Schmelzpunkt 144 °C - 145 °C.

Herstellung der Ausgangsverbindung

Beispiel II-1

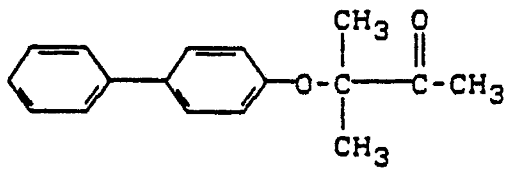

Zu einer Lösung von 129,2 g (1,5 Mol) 3-Methylbutan-2-on in 150 ml Tetrachlorkohlenstoff gibt man bei Raumtemperatur unter Rühren tropfenweise 76,3 ml (1.5 Mol) Brom, rührt nach beendeter Zugabe weitere 30 Minuten bei Raumtemperatur und engt dann im Vakuum ein.

Den Rückstand gibt man tropfenweise zu einer auf Rückflußtemperatur erhitzten Mischung von 255 g (1.5 Mol) 4-Hydroxybiphenyl und 248,8 g (1,8 Mol) Kaliumcarbonat in 400 ml Aceton, erhitzt nach beendeter Zugabe weitere 16 Stunden auf Rückflußtemperatur, läßt abkühlen, filtriert ungelöstes Kaliumcarbonat ab, wäscht mit Aceton nach und engt das Filtrat im Vakuum ein. Der Rückstand wird in Essigester aufgenommen, einmal mit kalter 1 normaler wässriger Natriumhydroxidlösung und zweimal mit Wasser gewaschen, über Natriumsulfat getrocknet, im Vakuum eingeengt und der Rückstand aus Ligroin umkristallisiert.

Man erhält 206,5 g (81,3 % der Theorie) an 3-[(1,1′-Biphenyl)-4-yloxy]-3-methylbutan-2-on als öligen Feststoff.

$^1$H-NMR ($CDCl_3$) : $\delta$ = 2,28 ppm (3 H).

Beispiel 2

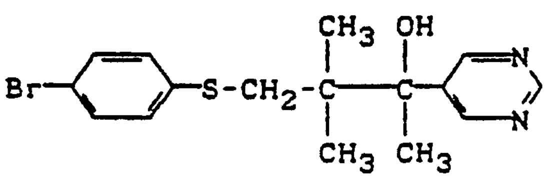

(Verfahren b)

Zu 7 g (0,02 Mol) 3-(4-Bromphenylthio)-2,2-dimethyl-1-(5-pyrimidinyl)-propan-1-on in 100 ml Ether gibt man bei Raumtemperatur tropfenweise unter Rühren 4,3 g (0,026 Mol) Methylmagnesiumiodid in 30 ml Ether und rührt nach beendeter Zugabe weitere 3 Stunden bei Raumtemperatur.

Zur Aufarbeitung wird mit Wasser versetzt, mit verdünnter Salzsäure auf pH 5-6 eingestellt, die organische Phase abgetrennt, mit Wasser gewaschen, über Natriumsulfat getrocknet, im Vakuun eingeengt und der Rückstand chromatographisch (Kieselgel; Essigester/Cyclohexan 3:1) gereinigt.

Man erhält 1,3 g (18 % der Theorie) an 4-(4-Bromphenylthio)-3,3-dimethyl-2-(5-pyrimidinyl)-butan-2-ol vom Schmelzpunkt 138 °C.

Herstellung der Ausgangsverbindung

Beispiel IV-1

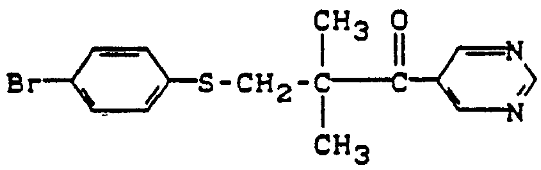

Zu 394,7 g (1.253 Mol) 5-(4-Bromphenylthio)-4,4-dimethyl-pent-1-en-1-ol-3-on in 2,2 l Tetrahydrofuran gibt man 67,7 g (1,58 Mol) Natriummethylat und 164,3 g (1.58 Mol) Formamidin Hydroacetat, rührt eine halbe Stunde bei Raumtemperatur, gibt dann tropfenweise unter Rühren 240 g (2.011 Mol) Dimethylformamid-dimethylacetal zu und rührt weitere sechs Stunden bei 60 °C.
Zur Aufarbeitung wird die erkaltete Reaktionsmischung abgesaugt, das Filtrat im Vakuum eingeengt und der Rückstand chromatographisch gereinigt.

Man erhält 90 g (20,5 % der Theorie) an 3-(4-Bromphenylthio)-2,2-dimethyl-1-(5-pyrimidinyl)-propan-1-on vom Schmelzpunkt 96 °C.

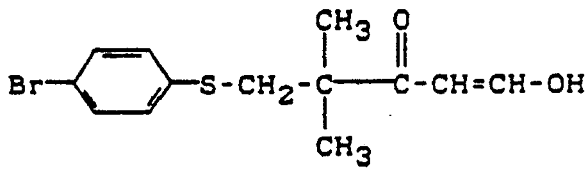

Zu 519 g (1.791 Mol) 4-(4-Bromphenylthio)-3,3-dimethylbutan-2-on und 96,7 g (1,791 Mol) Natriummethylat in 1,8 l Ether gibt man bei Raumtemperatur tropfenweise unter Rühren 132,6 g (1.791 Mol) Ameisensäureethylester und erhitzt anschließend 3 Stunden lang auf Rückflußtemperatur.
Zur Aufarbeitung versetzt man die abgekühlte Reaktionsmischung mit Wasser, trennt die organische Phase ab, wäscht die wässrige Phase zweimal mit Ether, stellt dann mit verdünnter Salzsäure auf pH 1 ein und extrahiert mehrfach mit Dichlormethan. Die vereinigten Dichlormethanphasen werden mit Wasser gewaschen, über Natriumsulfat getrocknet und im Vakuum eingeengt.

Man erhält 394.7 g (70 % der Theorie) an 5-(4-Bromphenylthio)-4,4-dimethyl-pent-1-en-1-ol-3-on als Öl.
$^1$H-NMR ($CDCl_3$/Tetramethylsilan):
$\delta$ = 1.36 (s, 6H); 3.15 (s, 2H) ppm.

Beispiel 3

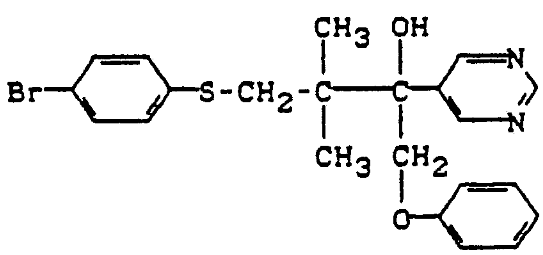

(Verfahren c)

10,6 g (0,029 Mol) 2-[1-(4-Bromphenylthio)-2-methylprop-2-yl]-2-(5-pyrimidinyl)-oxiran, 3,2 g (0.0335 Mol) Phenol und 12 g (0.0872 Mol) gemahlenes Kaliumcarbonat in 95 ml Dimethylformamid werden 2 Stunden bei 120 °C bis 130 °C gerührt, abgekühlt, in Wasser gegeben und mehrfach mit Dichlormethan extrahiert. Die vereinigten organischen Phasen werden mit Wasser gewaschen, über Natriumsulfat getrocknet, im Vakuum eingeengt und chromatographisch (Kieselgel; Essigester/Cyclohexan 3:1) gereinigt.

Man erhält 6,0 g (45 % der Theorie) an 4-(4-Bromphenylthio)-3,3-dimethyl-1-phenoxy-2-(5-pyrimidinyl)-butan-2-ol als Öl.
$^1$H-NMR ($CDCl_3$/Tetramethylsilan):
$\delta$ = 1.07 (s, 3H); 1.16 (s, 3H); 8,85 (s, 2H); 9.12 (s,1H) ppm.

Herstellung der Ausgangsverbindung

Beispiel VI-1

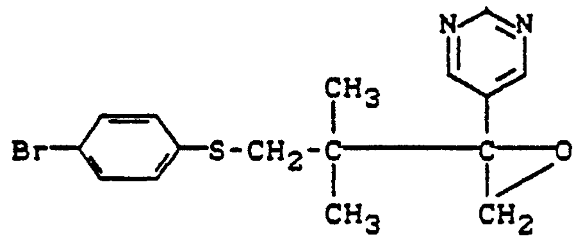

Zu einer Mischung aus 6,9 g (0.0312 Mol) Trimethylsulfoxoniumiodid, 7,9 g (0,0997 Mol) Dimethylsulfoxid und 3,6 g (0.0312 Mol) Kalium-t-butylat, die vorher 6 Stunden bei Raumtemperatur gerührt wurde, tropft man unter Rühren ebenfalls bei Raumtemperatur eine Lösung von 10 g (0.0285 Mol) 3-(4-Bromphenylthio)-2,2-dimethyl-1-(5-pyrimidinyl)-propan-1-on in 15 ml Tetrahydrofuran, rührt nach beendeter Zugabe weitere 15 Stunden bei Raumtemperatur, erhitzt dann 4 Stunden auf Rückflußtemperatur, kühlt ab, gießt die Mischung in Wasser, extrahiert mehrfach mit Essigester, wäscht die vereinigten organischen Phasen mit Wasser, trocknet über Natriumsulfat und entfernt das Lösungsmittel im Vakuum.

Man erhält 10,2 g (98 % der Theorie) an 2-[1-(4-Bromphenylthio)-2-methylprop-2-yl]-2-(5-pyrimidinyl)-oxiran als Öl, welches ohne weitere Reinigung in die nächste Reaktionsstufe eingesetzt wird.

In entsprechender Weise und gemäß den allgemeinen Angaben zur Herstellung erhält man die folgenden substituierten Pyrimidin-Derivate der allgemeinen Formel (I):

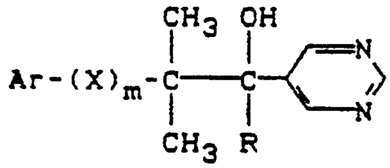

(I)

| Bsp. Nr. | Ar | $-(X)_m-$ | R | physikalische Eigenschaften |
|---|---|---|---|---|
| 4 | 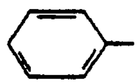 | $-CH_2-$ | $CH_3$ | $^1$H-NMR*); 0,78 (s); 0,92 (s) |
| 5 | 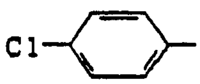 | -S- | $CH_3$ | Fp. 81-82 °C |
| 6 | 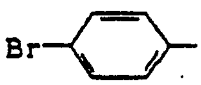 | $-CH_2-$ | $CH_3$ | $^1$H-NMR*): 1,72 (s); 8,85 (s); 9,07 (s) |
| 7 | 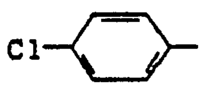 | $-CH_2-$ | $CH_3$ | Fp. 174 °C |

| Bsp. Nr. | Ar | -(X)$_m$- | R | physikalische Eigenschaften |
|---|---|---|---|---|
| 8 | 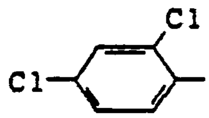 | -O- | $CH_3$ | $^1$H-NMR*): 1,25 (s); 1,36 (s); 1,85 (s) |
| 9 | 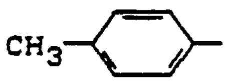 | -O- | $CH_3$ | $^1$H-NMR*): 1,14 (s); 1,20 (s); 1,80 (s); 2,31 (s) |
| 10 | 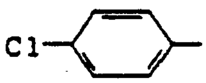 | -O- | $CH_3$ | Fp. 124 °C |
| 11 | 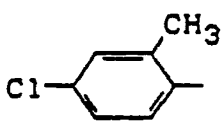 | -O- | $CH_3$ | Fp. 106 °C |
| 12 | 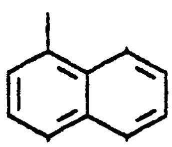 | -O- | $CH_3$ | Fp. 140 °C |
| 13 | 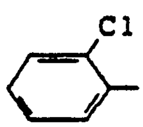 | $-CH_2-$ | $CH_3$ | $^1$H-NMR*): 2,07 (s) |
| 14 | 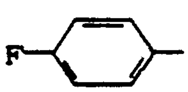 | $-CH_2-$ | $CH_3$ | $^1$H-NMR*): 2,05 (s) |

| Bsp. Nr. | Ar | $-(X)_m-$ | R | physikalische Eigenschaften |
|---|---|---|---|---|
| 15 | 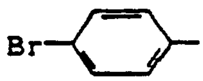 | -O- | $CH_3$ | Fp. 113 °C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurden die nachstehend aufgeführten Verbindungen als Vergleichsubstanzen eingesetzt:

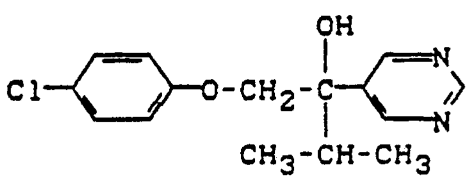

(A)

1-(4-Chlorphenoxy)-3-methyl-2-(5-pyrimidinyl)-butan-2-ol
(bekannt aus EP 131 867
und

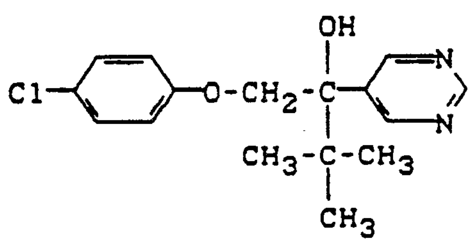

(B)

1-(4-Chlorphenoxy)-3,3-dimethyl-2-(5-pyrimidinyl)-butan-2-ol
(bekannt aus EP 1399).

Beispiel A

Pyrenophora teres-Test (Gerste) / protektiv

| | |
|---|---|
| Lösungsmittel: | 100 Gewichtsteile Dimethylformamid |
| Emulgator: | 0,25 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 5, 7, 10 und 12.

Beispiel B

Botrytis-Test (Bohne)/protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen 1 und 2.

Beispiel C

Venturia-Test (Apfel) / protektiv

| | |
|---|---|
| Lösungsmittel: | 4,7 Gewichtsteile Aceton |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann in Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1, 2 und 3.

## Ansprüche

1. Substituierte Pyrimidin-Derivate der allgemeinen Formel (I),

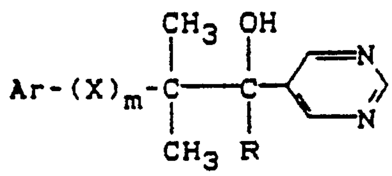

(I).

in welcher

Ar für gegebenenfalls substituiertes Aryl steht,

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,

wobei

n jeweils für eine Zahl 0, 1 oder 2 steht,

R für Alkyl, Alkenyl, Alkinyl, Alkoxyalkyl, Alkylthioalkyl, Cycloalkyl, Cycloalkylalkyl oder für jeweils gegebenenfalls substituiertes Aryl, Aralkyl, Aryloxyalkyl oder Arylthioalkyl steht und

m für eine Zahl 0 oder 1 steht,

sowie deren pflanzenverträgliche Säureadditionssalze und Metallsalzkomplexe.

2. Substituierte Pyrimidin-Derivate gemäß Anspruch 1, wobei in der Formel (I)

Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen oder jeweils gegebenenfalls einfach bis mehrfach, gleich oder verschieden durch Halogen substituiertes Phenyl oder Phenoxy;

oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;

X für Sauerstoff, Schwefel, Sulfinyl, Sulfonyl oder für eine der Gruppen $-CH_2-$; $-O-CH_2-$; $-CH_2-O-$; $-CH_2-O-$; $-O-CH_2-CH_2-$; $-S(O)_n-CH_2-$; $-CH_2-S(O)_n-$oder $-S(O)_n-CH_2-CH_2-$ steht,

wobei

n jeweils für eine Zahl 0, 1 oder 2 steht,

R für jeweils geradkettiges oder verzweigtes Alkyl mit 1 bis 6 Kohlenstoffatomen, Alkenyl mit 3 bis 5 Kohlenstoffatomen oder Alkinyl mit 3 bis 5 Kohlenstoffatomen, für jeweils geradkettiges oder verzweigtes Alkoxyalkyl oder Alkylthioalkyl mit jeweils 1 bis 6 Kohlenstoffatomen im Alkoxy-bzw. Alkylthioteil und jeweils 1 bis 3 Kohlenstoffatomen im Alkylteil, für Cycloalkyl mit 3 bis 7 Kohlenstoffatomen oder für Cycloalkylalkyl mit 3 bis 7 Kohlenstoffatomen im Cycloalkylteil und 1 bis 3 Kohlenstoffatomen im geradkettigen oder verzweigten Alkylteil oder für jeweils gegebenenfalls einfach bis mehrfach, gleich oder verschieden substituiertes Phenyl, Benzyl. Phenylethyl, Phenoxymethyl oder Phenylthiomethyl steht. wobei als Phenylsubstituenten jeweils die bei dem Rest Ar genannten infrage kommen und

m für eine Zahl 0 oder 1 steht.

3. Substituierte Pyrimidin-Derivate gemäß Anspruch 1, wobei in der Formel (I),
Ar für gegebenenfalls ein- bis mehrfach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Ethylthio, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Di chlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen oder jeweils gegebenenfalls ein-bis dreifach, gleich oder verschieden durch Fluor oder Chlor substituiertes Phenyl oder Phenoxy;
oder für gegebenenfalls ein- bis dreifach, gleich oder verschieden substituiertes α-Naphthyl oder β-Naphthyl steht, wobei als Substituenten jeweils infrage kommen: Fluor, Chlor, Brom, Cyano, Methyl, Ethyl, n- oder i-Propyl;
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
wobei
n jeweils für eine Zahl 0, 1 oder 2 steht,
R für Methyl, Ethyl, n- oder i-Propyl, n-, i-, s- oder t-Butyl, Allyl, Propargyl, für jeweils geradkettiges oder verzweigtes Alkoxymethyl oder Alkylthiomethyl mit jeweils 1 bis 4 Kohlenstoffatomen im Alkylteil, für Cyclopropyl, Cyclopentyl, Cyclohexyl, für Cyclopropylmethyl, Cyclopentylmethyl, Cyclohexylmethyl, Cyclopropylethyl, Cyclopentylethyl, Cyclohexylethyl oder für jeweils gegebenenfalls ein- bis dreifach, gleich oder verschieden substituiertes Phenyl, Benzyl, Phenylethyl, Phenoxymethyl oder Phenylthiomethyl steht, wobei als Phenylsubstituenten jeweils die bei dem Rest Ar genannten infrage kommen und
m für eine Zahl 0 oder 1 steht.

4. Verfahren zur Herstellung von substituierten Pyrimidin-Derivaten der allgemeinen Formel (I),

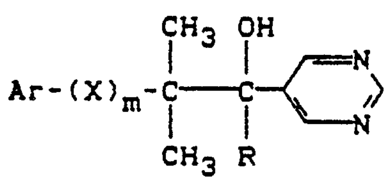

(I)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
wobei
n jeweils für eine Zahl 0, 1 oder 2 steht,
R für Alkyl, Alkenyl, Alkinyl, Alkoxyalkyl, Alkylthioalkyl, Cycloalkyl, Cycloalkylalkyl oder für jeweils gegebenenfalls substituiertes Aryl, Aralkyl, Aryloxyalkyl oder Arylthioalkyl steht und
m für eine Zahl 0 oder 1 steht,
sowie deren pflanzenverträgliche Säureadditionssalze und Metallsalzkomplexe, dadurch gekennzeichnet, daß man
(a) Ketone der Formel (II),

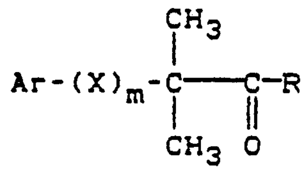

(II)

in welcher
Ar, X, R und m die oben angegebene Bedeutung haben,
mit 5-Lithiumpyrimidin der Formel (III)

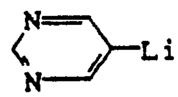

(III)

gegebenenfalls in Gegenwart eines Verdünnungsmittels umsetzt oder

(b) Pyrimidinylketone der Formel (IV),

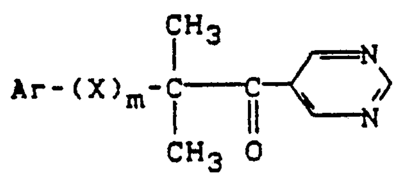

(IV)

in welcher
Ar, X und m die oben angegebene Bedeutung haben,
mit Alkyl-Grignard-Verbindungen der Formel (V),

R-Mg-Hal (V)

in welcher
R die oben angegebene Bedeutung hat und
Hal für Halogen steht,
gegebenenfalls in Gegenwart eines Verdünnungsmittels umsetzt oder daß man,

(c) um substituierte Pyrimidin-Derivate der Formel (Ia),

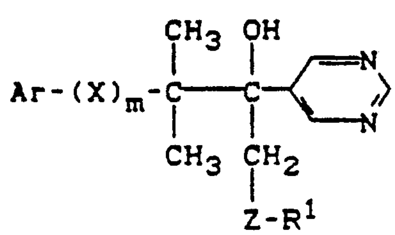

(Ia)

in welcher
Z für Sauerstoff oder Schwefel steht,
$R^1$ für Alkyl oder für gegebenenfalls substituiertes Aryl steht und
Ar, X und m die oben angegebenen Bedeutungen haben,
zu erhalten,
Pyrimidinyloxirane der Formel (VI),

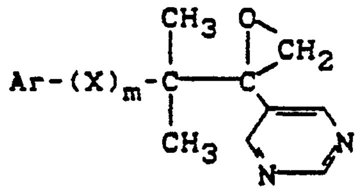

(VI)

in welcher
Ar, X und m die oben angegebene Bedeutung haben,
mit Alkoholen oder Thiolen der Formel (VII),
$R^1$-ZH (VII)
in welcher
$R^1$ und Z die oben angegebene Bedeutung haben,
gegebenenfalls in Gegenwart eines Verdünnungsmittels und gegebenenfalls in Gegenwart eines Reaktionshilfsmittel umsetzt und gegebenenfalls anschließend eine Säure oder ein Metallsalz addiert.

5. Schädlingsbekämpfungsmittel, gekennzeichnet durch einen Gehalt an mindestens einem substituierten Pyrimidin-Derivat der Formel (I) nach Ansprüchen 1 und 4.

6. Verwendung von substituierten Pyrimidin-Derivaten der Formel (I) nach den Ansprüchen 1 und 4 zur Bekämpfung von Schädlingen.

7. Verfahren zur Bekämpfung von Schädlingen, dadurch gekennzeichnet, daß man substituierte Pyrimidin-Derivate der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man substituierte Pyrimidin-Derivate der Formel (I) nach den Ansprüchen 1 und 4 mit Streckmitteln und/oder oberflächenaktiven Mitteln vermischt.

9. Pyrimidinyloxirane der Formel (VI),

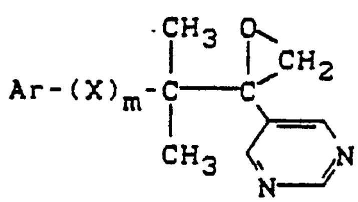

(VI)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S((O)_n-CH_2-CH_2-$ steht,
wobei
n jeweils für eine Zahl 0, 1 oder 2 steht und
m für eine Zahl 0 oder 1 steht.

10. Verfahren zur Herstellung von Pyrimidinyloxiranen der Formel (VI),

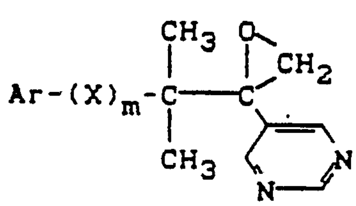

(VI)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,

wobei
n jeweils für eine Zahl 0, 1 oder 2 steht und
m für eine Zahl 0 oder 1 steht.
dadurch gekennzeichnet, daß man Pyrimidinylketone der Formel (IV),

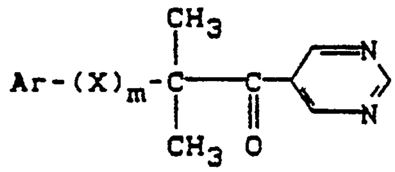

(IV)

in welcher
Ar, X und m die oben angegebene Bedeutung haben,
entweder a) mit Dimethyloxosulfonium-methylid der Formel (X)

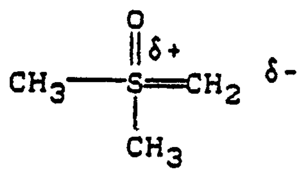

(X)

in Gegenwart eines Verdünnungsmittels bei Temperaturen zwischen 20 °C und 80 °C umsetzt oder b) mit Trimethylsulfoniummethylsulfat der Formel (XI)

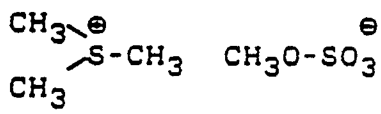

(XI)

in Gegenwart eines Verdünnungsmittels in Gegenwart einer Base bei Temperaturen zwischen 0 °C und 60 °C umsetzt.